(19) Europäisches Patentamt
European Patent Office
Office européen des brevets

(11) Veröffentlichungsnummer: **0 110 031**
**B1**

# (12) EUROPÄISCHE PATENTSCHRIFT

(45) Veröffentlichungstag der Patentschrift: **01.04.87**

(51) Int. Cl.⁴: **C 07 C 31/20**, C 07 C 43/13, C 07 C 29/00

(21) Anmeldenummer: **83108948.7**

(22) Anmeldetag: **10.09.83**

## (54) Verfahren zur Herstellung von 1,2-Diolen.

(30) Priorität: **19.11.82 DE 3242749**

(43) Veröffentlichungstag der Anmeldung: **13.06.84 Patentblatt 84/24**

(45) Bekanntmachung des Hinweises auf die Patenterteilung: **01.04.87 Patentblatt 87/14**

(84) Benannte Vertragsstaaten: **AT BE CH DE FR GB IT LI NL**

(56) Entgegenhaltungen:
**US - A - 2 086 239**
**US - A - 3 714 270**

**CHEMICAL ABSTRACTS, Band 92, Nr. 9, 3. März 1980, Seite 713, Nr. 76816r, Columbus, Ohio, US; A.S. SERIANNI u.a.: "Isotopically-enriched carbohydrates: the preparation of [2H]-enriched aldoses by catalytic hydrogenolysis of cyanohydrins with 2H2"**

(73) Patentinhaber: **Degussa Aktiengesellschaft, Weissfrauenstrasse 9, D-6000 Frankfurt am Main 1 (DE)**

(72) Erfinder: **Kleemann, Axel, Dr., Dipl.-Chem., Greifenhagenstrasse, D-6450 Hanau 9 (DE)**
Erfinder: **Deller, Klaus, Dr., Dipl.-Ing., Friedhofstrasse 47, D-6452 Hainburg (DE)**
Erfinder: **Drauz, Karlheinz, Dr., Dipl.-Chem., Flurstrasse 5, D-6463 Freigericht (DE)**
Erfinder: **Lehmann, Bernd, Dr., Dipl.-Chem., Ringstrasse 24 a, D-7750 Konstanz 19 (DE)**

EP 0 110 031 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein Verfahren zur Herstellung von 1,2-Diolen der allgemeinen Formel

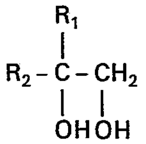
(I),

in der $R_1$ und $R_2$ gleich oder verschieden sind und jeweils Wasserstoff, einen geradkettigen oder verzweigten, unsubstituierten oder durch ein Halogenatom, eine Hydroxylgruppe, eine Methoxy- oder Ethoxygruppe oder einen Phenylrest substituierten Alkylrest mit 1 bis 10 Kohlenstoffatomen, einen Phenylrest oder einen Furylrest bedeuten oder $R_1$ und $R_2$ zusammen einen geradkettigen oder verzweigten Alkylenrest mit 2 bis 7 Kohlenstoffatomen bilden, welches dadurch gekennzeichnet ist, dass man ein Cyanhydrin der allgemeinen Formel

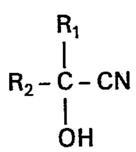
(II),

in der $R_1$ und $R_2$ die bereits genannte Bedeutung haben, in einem wasserhaltigen Medium, das pro Mol eingesetztem Cyanhydrin der allgemeinen Formel (II) mindestens 1 Mol Wasser enthält,

a) in Gegenwart eines Palladium- oder Platinkatalysators und, bezogen auf das Cyanhydrin der allgemeinen Formel (II), eines Äquivalents einer anorganischen oder organischen Säure oder mindestens eines Äquivalents eines sauren Ionenaustauschers oder in Gegenwart von metallischem Nickel und, wiederum bezogen auf das Cyanhydrin der allgemeinen Formel (II), mindestens eines Äquivalents eines sauren Ionenaustauschers bei einer Temperatur zwischen -20 und +25°C und einem Wasserstoffdruck von weniger als 10 bar hydriert, bis pro Mol eingesetztem Cyanhydrin der allgemeinen Formel (II) ein Mol Wasserstoff aufgenommen ist, und

b) in Gegenwart von metallischem Nickel bei einer Temperatur zwischen 30 und 100°C und einem Wasserstoffdruck zwischen 10 und 150 bar die Hydrierung bis zur Beendigung der Wasserstoffaufnahme fortführt.

Die als Ausgangsmaterialien für das erfindungsgemässe Verfahren dienenden Cyanhydrine der allgemeinen Formel (II) können in einfachster Weise nach bekannten Methoden (vgl. z.B. Houben-Weyl: Methoden der organischen Chemie, 4. Auflage, Georg Thieme Verlag, Stuttgart, Band VIII, Seiten 274 bis 278) aus den entsprechenden Aldehyden oder Ketonen durch Umsetzung mit Blausäure hergestellt werden. Sofern die Herstellung der Cyanhydrine in wässriger Lösung vorgenommen wird, können diese wässrigen Lösungen unmittelbar für das erfindungsgemässe Verfahren eingesetzt werden.

Die zur Herstellung der Cyanhydrine der allgemeinen Formel (II) benötigten Aldehyde oder Ketone ihrerseits können erforderlichenfalls auch in an sich bekannter Weise (vgl. z.B. Houben-Weyl: Methoden der organischen Chemie, 4. Auflage, Georg Thieme Verlag, Stuttgart, Band VII/1, Seiten 13 bis 503, bzw. Band VII/2a–c) hergestellt werden.

Beispiele für nach dem erfindungsgemässen Verfahren umzusetzende Cyanhydrine der allgemeinen Formel (II) sind Propionaldehydcyanhydrin, Isobutyraldehydcyanhydrin, Valeraldehydcyanhydrin, Hexanalcyanhydrin, Heptanalcyanhydrin, Octanalcyanhydrin, Butanoncyanhydrin, Pentanoncyanhydrin, Methylisopropylketoncyanhydrin, Diisopropylketoncyanhydrin, Cyclohexanoncyanhydrin, 2-Methylcyclohexanoncyanhydrin, 4-Hydroxy-4-methylpentanoncyanhydrin, Phenylacetaldehydcyanhydrin, Acetoncyanhydrin, Phenylacetoncyanhydrin, 5-Chlorpentanon-2-cyanhydrin, Methoxyacetaldehydcyanhydrin oder Mandelsäurenitril.

Besonders geeignet ist das erfindungsgemässe Verfahren für die Umsetzung von Glykolnitril zu Ethylenglykol, Milchsäurenitril (Acetaldehydcyanhydrin) zu 1,2-Propandiol oder n-Butyraldehydcyanhydrin zu 1,2-Pentandiol.

Die Cyanhydrine der allgemeinen Formel (II) werden in einem wasserhaltigen Medium, das pro Mol eingesetztem Cyanhydrin mindestens 1 Mol Wasser enthalten muss, hydriert. Sofern die Löslichkeit des eingesetzten Cyanhydrins dies zulässt, kann Wasser als alleiniges Lösungsmittel verwendet werden, sonst kommen auch Mischungen von Wasser mit wasserlöslichen Alkoholen, wie Methanol, Ethanol, n-Propanol, Isopropylalkohol, n-Butanol, Isobutylalkohol, sekundärer oder tertiärer Butylalkohol, Dioxan oder Tetrahydrofuran als Lösungsmittel in Frage. Das Lösungsmittel kann beispielsweise in einer Menge zwischen 0,5 und 100 ml, vorzugsweise zwischen 1 und 50 ml, pro Gramm eingesetztem Cyanhydrin angewandt werden.

In der Reaktionsstufe a) erfolgt die Hydrierung entweder in Gegenwart eines Palladium- oder Platinkatalysators oder in Gegenwart von metallischem Nickel. Geeignete Katalysatoren sind beispielsweise metallisches Palladium, insbesondere als Palladiummohr, metallisches Platin, insbesondere als Platinschwarz, oder Platin-IV-oxid. Wird metallisches Palladium oder Platin verwendet, so kann es in freier Form eingesetzt werden, aber auch in Form von Trägerkatalysatoren mit beispielsweise Aktivkohle, Bariumsulfat, Aluminiumoxid oder Siliciumdioxid als Trägermaterial. Besonders bevorzugte Katalysatoren sind Palladiummohr oder Palladium auf Aktivkohle. Das metallische Nickel wird vorzugsweise in der aktivierten Form des Raney-Nickels eingesetzt. Es können auch Gemische von mehreren Katalysatoren eingesetzt werden. Die eingesetzte Menge an Katalystor ist nicht kritisch, zur Erzielung kurzer Reaktionszeiten empfiehlt es sich jedoch, den Katalysator, berechnet als aktives Metall, in einer Menge zwischen 0,1 und 100, vorzugsweise zwischen 1 und 10, Gewichtsprozent, bezogen auf das eingesetzte Cyanhydrin der allgemeinen Formel (II), anzuwenden.

In der Reaktionsstufe a) ist ferner die Gegenwart einer Säure erforderlich. Bei Verwendung eines Palladium- oder Platinkatalysators kann dies eine organische Säure, wie Ameisensäure, Essigsäure oder Oxalsäure, sein. Bevorzugt werden jedoch Mineralsäuren, wie Schwefelsäure, Phosphorsäure und insbesondere Salzsäure, oder saure Ionenaustauscher verwendet. Die organischen oder anorganischen Säuren werden in der stöchiometrischen Menge von einem Äquivalent, bezogen auf das eingesetzte Cyanhydrin der allgemeinen Formel (II), zugesetzt. Die Anwendung eines Überschusses an Säure ist unzweckmässig. Die sauren Ionenaustauscher müssen in einer Menge von mindestens einem Äquivalent, bezogen auf das eingesetzte Cyanhydrin der allgemeinen Formel (II), zugesetzt werden. In diesem Falle stört jedoch ein Überschuss nicht. Wird als Hydrierungskatalysator metallisches Nickel verwendet, wird als Säure ein saurer Ionenaustauscher in einer Menge von ebenfalls wieder mindestens einem Äquivalent, bezogen auf das eingesetzte Cyanhydrin der allgemeinen Formel (II), zugesetzt.

Die Reaktionsstufe a) wird bei einer Temperatur zwischen -20 und +25°C, vorzugsweise zwischen 0 und 20°C, und bei einem Wasserstoffdruck von weniger als 10 bar, vorzugsweise zwischen 1 und 2 bar, durchgeführt. Besonders vorteilhaft ist es, wenn man einfach Wasserstoff durch das Reaktionsgemisch hindurchleitet. Nach Aufnahme von einem Mol Wasserstoff pro Mol eingesetztem Cyanhydrin der allgemeinen Formel (II) ist die Reaktionsstufe a) beendet.

In der sich nun anschliessenden Reaktionsstufe b) wird die Hydrierung in Gegenwart von metallischem Nickel, vorzugsweise in der aktivierten Form des Raney-Nickels, und unter schärferen Bedingungen fortgeführt. Es ist dabei nicht erforderlich, die im Reaktionsgemisch gegebenenfalls enthaltenen Palladium- oder Platinkatalysatoren und sauren Ionenaustauscher abzutrennen. Deren Abtrennung kann aber zweckmässig sein, um ihre Wiedergewinnung zu erleichtern. Wurde bereits die Reaktionsstufe a) in Gegenwart von metallischem Nickel durchgeführt, so ist im allgemeinen der Zusatz von weiterem Katalysator nicht erforderlich. Wurde dagegen die Reaktionsstufe a) in Gegegenwart eines Palladium- oder Platinkatalysators durchgeführt, so muss nun metallisches Nickel zugesetzt werden. Die zweckmässigen Mengen an Katalysator entsprechen den oben für die Reaktionsstufe a) angegebenen.

Die Reaktionsstufe b) wird bei einer Temperatur zwischen 30 und 100°C, vorzugsweise zwischen 45 und 70°C, und bei einem Wasserstoffdruck zwischen 10 und 150 bar, vorzugsweise zwischen 15 und 40 bar, durchgeführt.

Besonders hohe Ausbeuten an 1,2 Diolen der allgemeinen Formel (I) werden im allgemeinen erhalten, wenn die Reaktionsstufe a) in Gegenwart eines Palladiumkatalysators durchgeführt wird.

Nach beendeter Wasserstoffaufnahme werden die im Reaktionsgemisch enthaltenen Katalysatoren und gegebenenfalls Ionenaustauscher beispielsweise durch Filtration oder Zentrifugation, abgetrennt. Aus der verbleibenden Lösung kann dann das enthaltene 1,2-Diol in an sich bekannter Weise, z.B. durch fraktionierte Destillation oder durch Extraktion mit einem geeigneten organischen Lösungsmittel und anschliessende fraktionierte Destillation, isoliert werden.

Die Erfindung wird durch die nachfolgenden Beispiele näher erläutert. Prozentangaben bedeuten, sofern nicht anders angegeben, Gewichtsprozente.

Beispiel 1

30,2 g (0,5 mol) Glykolnitril (94,3%ig) werden in 150 ml $H_2O$ gelöst und 300 ml eines sauren Ionenaustauschers zugegeben.

Nach Zugabe von 3,5 g eines 10% Pd/Aktivkohle-Katalysators wird unter Rühren bei 15°C und einem $H_2$-Druck von 2 bar bis zur Aufnahme von 0,5 mol Wasserstoff hydriert. Danach werden 1,5 g eines aktivierten Ni-Katalysators zugegeben, die Temperatur auf 50°C und der Druck auf 15 bar erhöht und bis zum Ende der $H_2$-Aufnahme weiterhydriert. Dann wird von den Katalysatoren und vom Ionenaustauscher abfiltriert. Die Lösung wird am Rotationsverdampfer eingeengt und destilliert.

Der Hauptlauf (26,2 g) geht bei 196 bis 198°C über. $n_D^{20}$ = 1,4310. Ausbeute an Ethylenglykol: 84% der Theorie.

Beispiel 2

24,2 g (0,4 mol) Glykolnitril (94,3%ig) werden in 200 ml $H_2O$ gelöst und mit 40 g NCl konzentriert versetzt. Nach Zugabe von 2,5 g eines 10% Pd/Aktivkohle-Katalysators wird unter Rühren bei 20°C und 5 bar Wasserstoffdruck bis zur Aufnahme von 0,4 mol Wasserstoff hydriert. Danach werden 2 g eines aktivierten Ni-Katalysators zugegeben, die Temperatur auf 30°C und der Wasserstoffdruck auf 15 bar erhöht und bis zum Ende der Wasserstoffaufnahme weiterhydriert. Dann wird von den Katalysatoren abfiltriert. Die Lösung wird am Rotationsverdampfer eingeengt und destilliert. Der Hauptlauf (20,6 g) geht bei 196 bis 198°C über. $n_D^{20}$ = 1,4310. Ausbeute an Ethylenglykol: 82,6% der Theorie.

Beispiel 3

40,0 g (0,5 mol) Acetaldehydcyanhydrin werden in 200 ml $H_2O$ gelöst und 300 ml eines sauren Ionenaustauschers zugegeben. Nach Zugabe von 3,5 g eines 10% Pd/Aktivkohle-Katalysators wird unter Rühren bei 15°C und einem $H_2$-Druck von 2 bar bis zur Aufnahme von 0,5 mol Wasserstoff

hydriert. Danach werden 1,5 g eines aktivierten Ni-Katalysators zugegeben, die Temperatur auf 50°C und der Druck auf 15 bar erhöht und bis zum Ende der $H_2$-Aufnahme weiterhydriert. Dann wird von den Katalysatoren und vom Ionenaustauscher abilftriert. Die Lösung wird am Rotationsverdampfer eingeengt und destilliert.

Der Hauptlauf (28,1 g) geht bei 16 mbar/84°C über. $n_D^{20}$ = 1,4320. Ausbeute 74% der Theorie an 1,2-Propandiol.

Beispiel 4

35 g (0,4 mol) Acetoncyanhydrin werden in 250 ml $H_2O$ gelöst und 250 ml eines sauren Ionenaustauschers zugegeben. Nach Zugabe von 3,5 g eines 10% Pd/Aktivkohle-Katalysators wird unter Rühren bei 15°C und einem $H_2$-Druck von 2 bar bis zur Aufnahme von 0,5 mol Wasserstoff hydriert. Danach werden 1,5 g eines aktivierten Ni-Katalysators zugegeben, die Temperatur auf 50°C und der Druck auf 15 bar erhöht und bis zum Ende der $H_2$-Aufnahme weiterhydriert. Dann wird von den Katalysatoren und vom Ionenaustauscher abfiltriert. Die Lösung wird am Rotationsverdampfer eingeengt und destilliert.

Der Hauptlauf (29,3 g) geht bei 16 mbar/79°C über. $n_D^{20}$ = 1,4360. Ausbeute an 2-Methyl-1,2-propandiol: 81% der Theorie.

| Elementaranalyse | %C | % H |
|---|---|---|
| Berechnet: | 53,31 | 11,19 |
| Gefunden: | 53,23 | 11,02 |

Beispiel 5

42,0 g (300 mmol) Mandelsäurenitril werden in 220 ml $H_2O$ und 130 ml Ethanol gelöst und 150 ml eines sauren Ionenaustauschers (Lewatit S 100) zugegeben. Nach Zugabe von 4,0 g eines 10% Pd/Aktivkohle-Katalysators wird unter Rühren bei 15°C und einem $H_2$-Druck von 1,5 bar bis zur Aufnahme von 0,3 mol $H_2$ hydriert. Danach werden 2 g eines aktivierten Ni-Katalysators zugegeben, die Temperatur auf 45°C und der Druck auf 15 bar erhöht und bis zum Ende der Wasserstoffaufnahme weiterhydriert. Dann wird von den Katalysatoren und vom Ionenaustauscher abfiltriert. Die Reaktionslösung wird am Rotationsverdampfer eingeengt. Der Rückstand sind farblose Kristalle, die aus Ethanol umkristallisiert werden.

Ausbeute an 1-Phenyl-1,2-ethandiol 25,1 g, entsprechend 61% der Theorie.

Schmelzpunkt: 65°C.

Beispiel 6

40,0 g (0,4 mol) Butyraldehydcyanhydrin werden in 250 ml $H_2O$ und 60 ml Ethanol gelöst und 200 ml eines sauren Ionenaustauschers (Lewatit S 100) zugegeben. Nach Zugabe von 3 g eines 10% Pd/Aktivkohle-Katalysators wird unter Rühren bei 15°C und einem $H_2$-Druck von 1,2 bar bis zur Aufnahme von 0,4 mol Wasserstoff hydriert. Danach werden 3 g eines aktivierten Ni-Katalysators zugegeben und die Temperatur auf 50°C und der Wasserstoffdruck auf 15 bar erhöht und bis zum Ende der Wasserstoffaufnahme weiterhydriert. Danach wird von den Katalysatoren und vom Ionenaustauscher abfiltriert. Die 1,2-Pentandiolabtrennung erfolgt destillativ. Der Hauptlauf geht bei 53 bis 55°C/0,06 mbar über. Ausbeute an 1,2-Pentandiol: 31,7 g, entsprechend 77% der Theorie.

Beispiel 7

50,0 g (0,5 mol) Butyraldehydcyanhydrin werden in 100 ml Ethanol und 350 ml $H_2O$ gelöst und 300 ml eines sauren Ionenaustauschers (Lewatit S 100) zugegeben. Nach Zugabe von 4 g eines aktivierten Ni-Katalysators wird unter Rühren bei 15°C und einem Wasserstoffdruck von 1,2 bar bis zur Aufnahme von 0,5 mol Wasserstoff hydriert. Danach wird der Wasserstoffdruck auf 15 bar und die Temperatur auf 50°C erhöht und bis zum Ende der Wasserstoffaufnahme weiterhydriert. Dann wird vom Katalysator und Ionenaustauscher abfiltriert. Die Lösung wird am Rotationsverdampfer eingeengt, danach destilliert. Der Hauptlauf geht bei 53 bis 55°C und 0,06 mbar über. Ausbeute an 1,2-Pentandiol: 35,5 g, entsprechend 68,2 g der Theorie.

Beispiel 8

24,8 g (0,25 mol) Butyraldehydcyanhydrin werden in 180 ml $H_2O$ und 20 ml Ethanol gelöst und mit 25 g HCl konzentriert versetzt. Nach Zugabe von 1,25 g eines 10% Pd/Aktivkohle-Katalysators wird unter Rühren bei 25°C und 9 bar Wasserstoffdruck bis zur Aufnahme von 0,25 mol Wasserstoff hydriert. Danach werden 2,0 g eines aktivierten Ni-Katalysators zugegeben, die Temperatur auf 35°C und der Wasserstoffdruck auf 20 bar erhöht und bis zum Ende der Wasserstoffaufnahme weiterhydriert. Die Lösung wird am Rotationsverdampfer eingeengt und destilliert. Der Hauptlauf (19,1 g) geht bei 53 bis 55°C/0,06 mbar über. Ausbeute an 1,2-Pentandiol: 73,3% der Theorie.

Beispiel 9

Beispiel 8 wird wiederholt, mit dem einzigen Unterschied, dass statt HCl konzentriert 13 g $H_2SO_4$ eingesetz werden. Ausbeute an 1,2-Pentandiol: 74% der Theorie.

Beispiel 10

Beispiel 8 wird wiederholt, mit dem einzigen Unterschied, dass statt HCl konzentriert 15 g Eisessig eingesetzt werden.

Ausbeute an 1,2-Pentandiol: 70% der Theorie.

Beispiel 11

Beispiel 8 wird wiederholt, mit dem einzigen Unterschied, dass statt eines Pd/Aktivkohle-Katalysators ein 10% Pt/Aktivkohle-Katalysator eingesetzt wird.

Ausbeute an 1,2-Pentandiol: 65% der Theorie.

Beispiel 12

Beispiel 8 wird wiederholt, mit dem einzigen

Unterschied, dass statt eines Pd/Aktivkohle-Katalysators Pd-Mohr eingesetzt wird.

Ausbeute an 1,2-Pentandiol: 72% der Theorie.

Beispiel 13

Beispiel 8 wird wiederholt, mit dem einzigen Unterschied, dass statt eines Pd/Aktivkohle-Katalysators ein 10% $Pd/BaSO_4$-Katalysator eingesetzt wird.

Ausbeute an 1,2-Pentandiol: 75% der Theorie.

Beispiel 14

Beispiel 8 wird wiederholt, mit dem einzigen Unterschied, dass statt eines Pd/Aktivkohle-Katalysators ein 10% $Pd/SiO_2$-Katalysator eingesetzt wird.

Ausbeute an 1,2-Pentandiol: 71% der Theorie.

**Patentansprüche**

1. Verfahren zur Herstellung von 1,2-Diolen der allgemeinen Formel

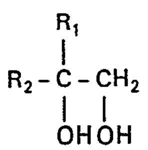

(I),

in der $R_1$ und $R_2$ gleich oder verschieden sind und jeweils Wasserstoff, einen geradkettigen oder verzweigten, unsubstituierten oder durch ein Halogenatom, eine Hydroxylgruppe, eine Methoxy- oder Ethoxygruppe oder einen Phenylrest substituierten Alkylrest mit 1 bis 10 Kohlenstoffatomen, einen Phenylrest oder einen Furylrest bedeuten oder $R_1$ und $R_2$ zusammen einen geradkettigen oder verzweigten Alkylenrest mit 2 bis 7 Kohlenstoffatomen bilden, dadurch gekennzeichnet, dass man ein Cyanhydrin der allgemeinen Formel

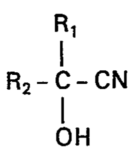

(II),

in der $R_1$ und $R_2$ die bereits genannte Bedeutung haben, in einem wasserhaltigen Medium, das pro Mol eingesetztem Cyanhydrin der allgemeinen Formel (II) mindestens 1 Mol Wasser enthält,

a) in Gegenwart eines Palladium- oder Platinkatalysators und, bezogen auf das Cyanhydrin der allgemeinen Formel (II), eines Äquivalents einer anorganischen oder organischen Säure oder mindestens eines Äquivalents eines sauren Ionenaustauschers oder in Gegenwart von metallischem Nickel und, wiederum bezogen auf das Cyanhydrin der allgemeinen Formel (II), mindestens eines Äquivalents eines sauren Ionenaustauschers bei einer Temperatur zwischen −20 und +25°C und einem Wasserstoffdruck von weniger als 10 bar hydriert, bis pro Mol eingesetztem Cyanhydrin der allgemeinen Formel (II) ein Mol Wasserstoff aufgenommen ist, und

b) in Gegenwart von metallischem Nickel bei einer Temperatur zwischen 30 und 100°C und einem Wasserstoffdruck zwischen 10 und 150 bar die Hydrierung bis zur Beendigung der Wasserstoffaufnahme fortführt.

2. Verfahren nach Anspruch 1, dadurch gekennzeichnet, dass man die Reaktionsstufe a) in Gegenwart eines Palladium- oder Platinkatalysators und von Salzsäure durchführt.

3. Verfahren nach Anspruch 1 oder 2, dadurch gekennzeichnet, dass man die Reaktionsstufe a) bei einer Temperatur zwischen 0 und 20°C durchführt.

4. Verfahren nach einem der Ansprüche 1 bis 3, dadurch gekennzeichnet, dass man die Reaktionsstufe a) bei einem Wasserstoffdruck zwischen 1 und 2 bar durchführt.

5. Verfahren nach einem der Ansprüche 1 bis 4, dadurch gekennzeichnet, dass man die Reaktionsstufe b) bei einer Temperatur zwischen 45 und 70°C durchführt.

6. Verfahren nach einem der Ansprüche 1 bis 5, dadurch gekennzeichnet, dass man die Reaktionsstufe b) bei einem Wasserstoffdruck zwischen 15 und 40 bar durchführt.

7. Verfahren nach einem der Ansprüche 1 bis 6, dadurch gekennzeichnet, dass man die Verfahrensstufen a) und b) in Wasser oder in einem Gemisch aus Wasser und einem wasserlöslichen Alkohol, Dioxan oder Tetrahydrofuran druchführt.

**Claims**

1. A process for the production of 1,2-diols corresponding to the general formula

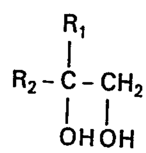

(I),

in which $R_1$ and $R_2$ are the same or different and each represent hydrogen, a straight-chained or branched alkyl radical containing from 1 to 10 carbon atoms which is unsubstituted, or substituted by a halogen atom, a hydroxyl group, a methoxy or ethoxy group or a phenyl radical, a phenyl radical or a furyl radical, or $R_1$ and $R_2$ together form a straight-chained or branched alkylene radical containing from 2 to 7 carbon atoms, characterised in that a cyanohydrin corresponding to the general formula

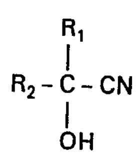

(II),

in which $R_1$ and $R_2$ have the meaning already given, is hydrogenated in a water-containing medium containing at least 1 mol of water per mol of used cyanohydrin corresponding to the general formula (II),

a) in the presence of a palladium or platinum catalyst and, with respect to the cyanohydrin corresponding to general formula (II), of an equivalent of an inorganic or organic acid or of at least one equivalent of an acidic ion exchanger or in the presence of metallic nickel and, again with respect to the cyanohydrin corresponding to general formula (II), of at least one equivalent of an acid ion exchanger at a temperature of between -20 and +25°C and at a hydrogen pressure of less than 10 bars until 1 mol of hydrogen is taken up per mol of used cyanohydrin corresponding to the general formula (II), and

b) hydrogenation is continued in the presence of metallic nickel at a temperature of between 30 and 100°C and at a hydrogen pressure of between 10 and 150 bars until termination of hydrogen uptake.

2. A process according to claim 1, characterised in that the reaction stage a) is carried out in the presence of a palladium or platinum catalyst and of hydrochloric acid.

3. A process according to claim 1 or 2, characterised in that the reaction stage a) is carried out at a temperature of between 0 and 20°C.

4. A process according to one of claims 1 to 3, characterised in that the reaction stage a) is carried out at a hydrogen pressure of between 1 and 2 bars.

5. A process according to one of claims 1 to 4, characterised in that the reaction stage b) is carried out at a temperature of between 45 and 70°C.

6. A process according to one of claims 1 to 5, characterised in that the reaction stage b) is carried out at a hydrogen pressure of between 15 and 40 bars.

7. A process according to one of claims 1 to 6, characterised in that the process stages a) and b) are carried out in water or in a mixture of water and a water-soluble alcohol, dioxan or tetrahydrofuran.

**Revendications**

1. Procédé pour la fabrication de diols-1,2, répondant à la formule générale:

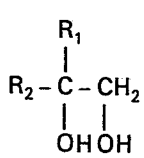

(I),

où $R_1$ et $R_2$ sont semblables ou différents, et représentent chaque fois un hydrogène, un reste alcoyl à 1 à 10 atomes de carbone, à chaine linéaire ou ramifiée, non substitué, ou substitué par un atome d'halogène, un groupe hydroxyl, un groupe méthoxy ou éthoxy ou un reste phényl, un reste phényl ou un reste furyl, ou $R_1$ et $R_2$ forment en commun un reste alcoylene à chaine droite ou ramifiée à 2 à 7 atomes de carbone, procédé caractérisé en ce que l'on hydrogène une cyanhydrine répondant à la formule générale:

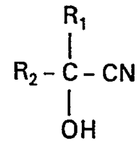

(II),

où $R_1$ et $R_2$ ont la signification déjà indiquée, dans un milieu contenant de l'eau, qui contient, par mole de cyanhydrine de formule générale (II) mise en oeuvre, au moins une mole d'eau,

a) en présence d'un catalyseur de palladium ou de platine, et, en calculant sur la cyanhydrine de formule générale (II), un équivalent d'un acide minéral ou organique ou d'au moins un équivalent d'un échangeur d'ions acide, ou en présence de nickel métal, et en calculant à nouveau sur la cyanhydrine de formule générale (II) mise en oeuvre, au moins un équivalent d'un échangeur d'ions acide, à une température qui se situe entre -20 et +25°C, et sous une pression d'hydrogène inférieure à 10 bars, jusqu'à ce qu'il soit absorbé une mole d'hydrogène par mol de cyanhydrine de formule générale (II) mise en oeuvre, et

b) on poursuit l'hydrogénation, en présence de nickel métal, à une température qui se situe entre 30 et 100°C et sous une pression d'hydrogène entre 10 et 150 bars jusqu'à ce que l'absorption d'hydrogène soit terminée.

2. Procédé suivant la revendication 1, caractérisé en ce qu'on effectue l'étape de réaction a) en présence d'un catalyseur de palladium ou de platine, et d'acide chlorhydrique.

3. Procédé suivant l'une des revendications 1 et 2, caractérisé en ce que l'on effectue l'étape de réaction a) à une température située entre 0 et 20°C.

4. Procédé suivant l'une des revendications 1 à 3, caractérisé en ce que l'on effectue l'étape de réaction a) sous une pression d'hydrogène située entre 1 et 2 bars.

5. Procédé suivant l'une des revendications 1 à 4, caractérisé en ce que l'on effectue l'étape de réaction b) à une température située entre 45 et 70°C.

6. Procédé suivant l'une des revendications 1 à 5, caractérisé en ce que l'on exécute l'étape de réaction b) sous une pression d'hydrogène située entre 15 et 40 bars.

7. Procédé suivant l'une des revendications 1 à 6, caractérisé en ce que l'on exécute les étapes d'opération a) et b) dans l'eau, ou dans un mélange d'eau et d'un alcool soluble à l'eau, de dioxane ou de tétrahydrofuranne.